# EUROPEAN PATENT APPLICATION

(11) **EP 3 326 566 A1**
(43) Date of publication of application: **30.05.2018**
(21) Application number: 16827863.8
(22) Date of filing: 22.07.2016
(51) Int. Cl.: A61B 34/35

(54) **MEDICAL SYSTEM AND OPERATION METHOD THEREFOR**

(30) Priority: 23.07.2015 US 201562195869 P
(71) Applicant: Olympus Corporation, Hachioji-shi, Tokyo 192-8507 (JP)
(72) Inventor: INOUE Shintaro, Hachioji-shi Tokyo 192-8507 (JP)
(74) Representative: Gunzelmann, Rainer
(86) International application number: PCT/JP2016/071586
(87) International publication number: WO 2017/014303

(57) **Abstract**

A medical system (1) includes: a distal end having an imager (5) and a plurality of joints; a drive part (15) configured to generate power for operating the joints; and a controller (51) configured to control the drive part (15), and the controller (51) includes: a characteristic point setting part (53) configured to extract characteristic points of an object and recognizes the object based on the characteristic points; a distance measurement part (54) configured to measure the distance between the imager (5) and the object; a correction amount calculation part (63) configured to calculate the amount of operation of the joints such that the imager (5) is directed to the object and adjust the distance between the imager (5) and the object to a predetermined distance; and a drive signal generation part (65) configured to generate a drive signal for operating the drive part (15) based on the amount of operation and output the drive signal to the drive part (15).

## Description

### [Technical Field]

The present invention relates to a medical system and an operation method therefor.

Priority is claimed on U.S. Provisional Patent Application No. 62/195,869, filed on July 23, 2015, the content of which is incorporated herein by reference.

### [Background Art]

Medical systems for surgery and the like in the body are widely known.

For example, Patent Document 1 discloses the use of an endoscopic treatment instrument inserted in a channel of a flexible endoscope having an imager. The flexible endoscope disclosed in Patent Document 1 has a knob for manually adjusting the orientation of the distal portion of the flexible endoscope. According to the technology disclosed in Patent Document 1, by manually operating the knob of the endoscope, the imager and the endoscopic treatment instrument can be set to an arbitrary orientation within the movable range of the imager.

In addition, Patent Document 2 discloses a medical system that can automatically adjust the angle of the end effector so that the end effector faces the reference point set for the target. In the technology disclosed in Patent Document 2, since the operation of directing the end effector to the reference point is automated, the burden on the operator is reduced.

### [Citation List]

### [Patent Document]

[Patent Document 1]
   Japanese Unexamined Patent Application, First Publication No. 2009-195489
[Patent Document 2]
   Japanese Unexamined Patent Application, First Publication No. 2015-24026

### [Summary of Invention]

### [Technical Problem]

In the technology disclosed in Patent Document 1, when an attempt is made to keep the distance between the target and the imager constant in a state in which the imager faces the target, it is very burdensome for the operator of the endoscope. Also, with the technology disclosed in Patent Document 2, the operator has to manually adjust the distance between the end effector and the target.

The present invention has been made in view of the above-described problems, and it is an object of the present invention to provide a medical system and an operating method therefor that can be automatically adjusted so as to have a preferable positional relationship with a target within a movable range of a medical system.

### [Solution to Problem]

One aspect of the present invention is a medical system including: a distal end having an end effector and a plurality of joints that changes an orientation and a position of the end effector; a drive part configured to generate power for operating the joints; and a controller configured to control the drive part, wherein the controller includes: a characteristic point setting part configured to extract a characteristic point of a target object and recognize the target object based on the characteristic point; a distance measurement part configured to measure a distance between the end effector and the target object; a correction amount calculator configured to calculate an amount of operation of the joints such that the end effector is directed to the target object and adjust the distance between the end effector and the target object to a predetermined distance; and a drive signal generator configured to generate a drive signal for operating the drive part based on the amount of operation and output the drive signal to the drive part.

The medical system according to the above aspect may further include: a force detector that is connected to the controller and detects a force which the joints receive from outside, wherein the controller may further include an external force determinator configured to determine whether or not a force externally applied to the joints exceeds a predetermined value, and the correction amount calculator may generate a correction command for reducing the distance between the end effector and the target to be shorter than the predetermined distance when it is determined by the external force determinator that the force exceeds the predetermined value.

The medical system according to the above aspect may further include: a force detector that is connected to the controller and detects a force which the joints receive from outside, wherein the distal end may have three or more of the joints, the controller may further include a joint specifying part that specifies a joint subjected to a force exceeding a predetermined value among the plurality of joints, and the correction amount calculator may generate a correction command for moving the joint specified by the joint specifying part such that the force applied to the joint specified by the joint specifying part is equal to or less than the predetermined value and the position and the orientation of the end effector are maintained.

The end effector may have an imager that images the target object, the characteristic point setting part may recognize the target object by using an image imaged by the imager, and the correction amount calculator may calculate an amount of operation of the drive part so that the target object is located at an image center of the imager.

The imager may be capable of imaging a stereo image of the target object, and the distance measurement part may calculate a distance between the target object and the imager using the stereo image.

The medical system according to above aspect may further include: an operation part configured to give an operation command to the controller, wherein the operation part may include: a master arm including an input part corresponding to the end effector and a plurality of master joints corresponding to the joints and having a shape conforming to the distal end; and a master drive part that is connected to the controller and controls an operation of the master joint, the controller may further include an operation command generator configured to detect an amount of operation of the master joint and generate an operation command including an amount of operation of a corresponding joint, and the drive signal generator may output a drive signal to the master drive part so that the joint and the master arm maintain a similarity relationship.

The distal end may have a channel through which a medical instrument can be inserted.

The medical system according the above aspect may further include: an insertion state determination mechanism provided in the channel so as to determine whether or not a treatment tool that can be passed through the channel is inserted through the channel; an insertion state detector configure to obtain a second predetermined distance preset corresponding to a type of the treatment tool, based on a determination state by the insertion state determination mechanism; and a control target value setting part configured to set a control target value of a distance between the end effector and the target object to the second predetermined distance, wherein the correction amount calculator may calculate the amount of operation of the joints so that the distance between the end effector and the target object becomes the second predetermined distance.

Another aspect of the present invention is a method of operating a medical system including a distal end having an end effector disposed thereon, and the method includes: a characteristic point recognition step of extracting a characteristic point of a target object and recognizing the target object based on the characteristic point; a distance measuring step of measuring a distance between the end effector and the target object; and a correction amount calculation step of calculating an amount of operation of joints so that the end effector is directed to the target object and the distance between the end effector and the target object becomes a predetermined distance.

### [Advantageous Effects of Invention]

According to the present invention, it is possible to automatically adjust the position and orientation of the end effector so as to have a preferable positional relationship with respect to the target within the movable range of the medical system.

### [Brief Description of Drawings]

FIG. 1 is an overall view of a medical system according to a first embodiment of the present invention.
FIG. 2 is a partial cross-sectional view of a manipulator of the medical system.
FIG. 3 is a schematic diagram showing an operation input device of the medical system.
FIG. 4 is a block diagram of a main part of the medical system.
FIG. 5 is a block diagram of a main part of the medical system.
FIG. 6 is a flowchart for explaining a control procedure in a controller of the medical system.
FIG. 7 is a diagram for explaining the operation of the medical system.
FIG. 8 is a diagram for explaining the operation of the medical system.
FIG. 9 is a diagram for explaining the operation of the medical system.
FIG. 10 is a diagram for explaining the operation of the medical system.
FIG. 11 is an overall view of a medical system according to a second embodiment of the present invention.
FIG. 12 is a block diagram of a main part of the medical system.
FIG. 13 is a flowchart for explaining a control procedure in a controller of the medical system.
FIG. 14 is a diagram for explaining the operation of the medical system.
FIG. 15 is a diagram for explaining the operation of the medical system.
FIG. 16 is a schematic diagram showing a part of a manipulator of a medical system of a modified example of the second embodiment.
FIG. 17 is a block diagram of a main part of the medical system.
FIG. 18 is a flowchart showing a control procedure in a controller of the medical system.
FIG. 19 is a diagram for explaining the operation of the medical system.

### [Description of Embodiments]

### (First Embodiment)

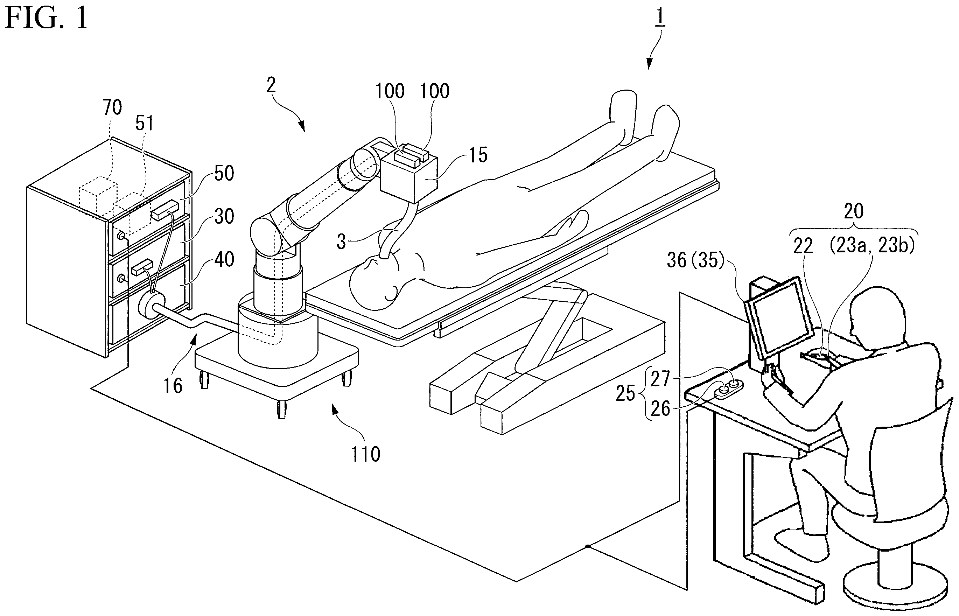

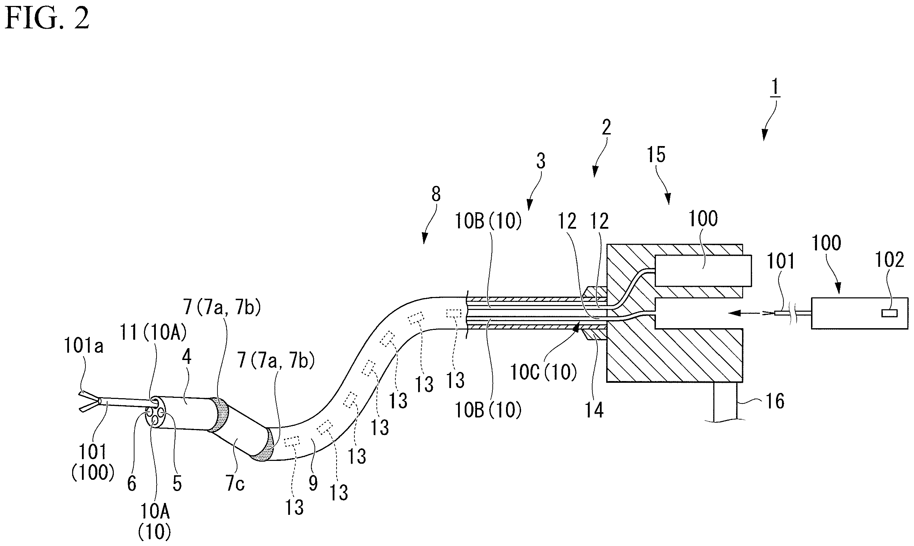

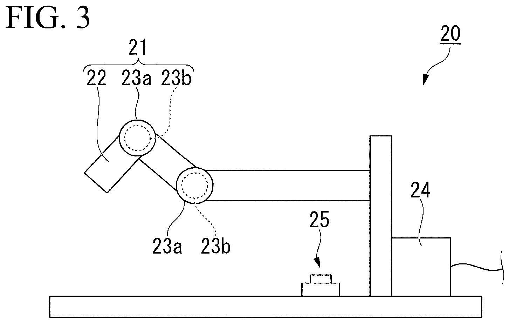

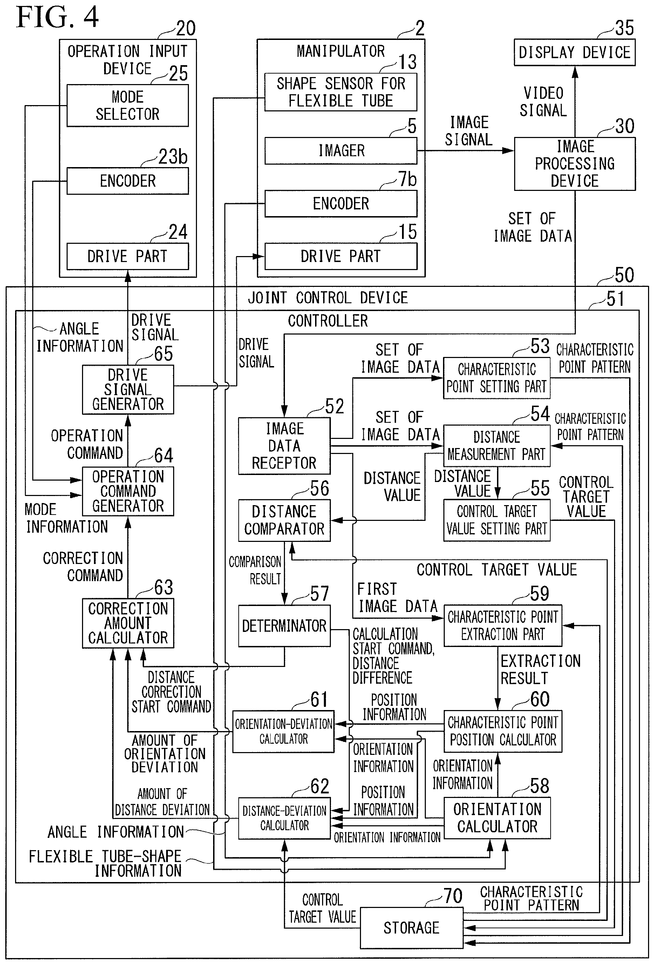

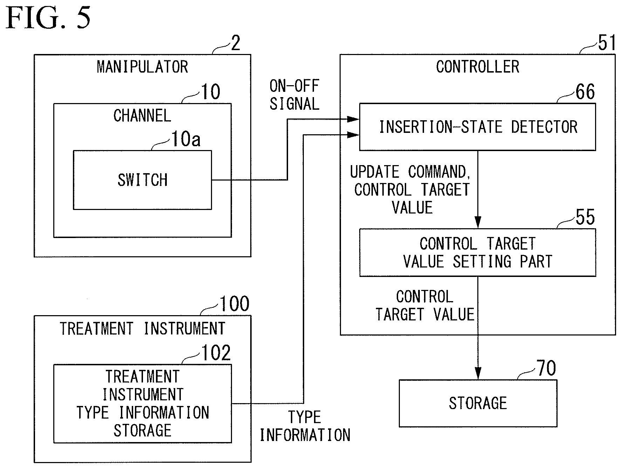

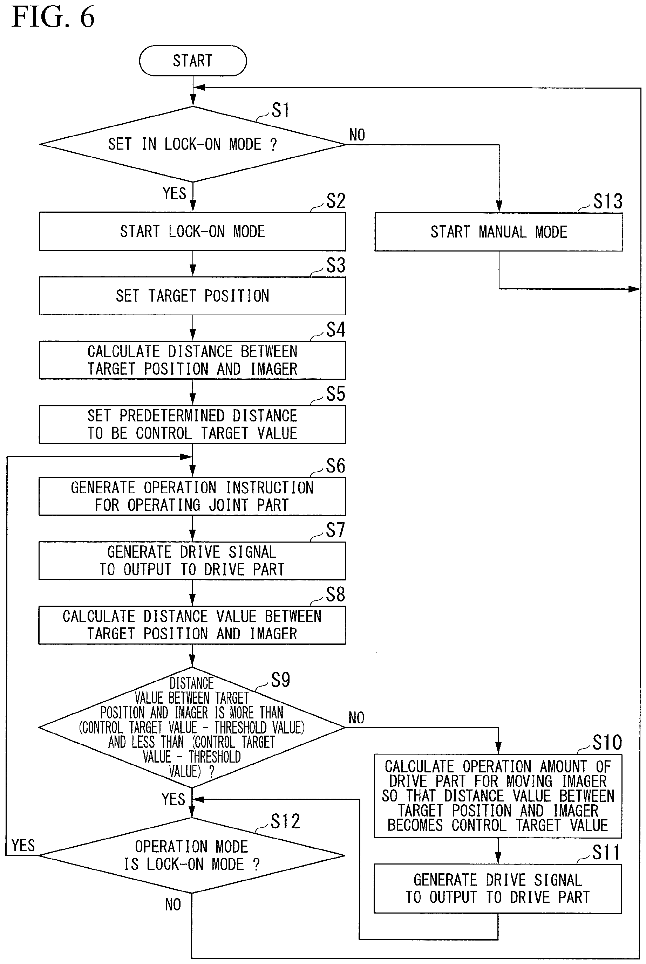

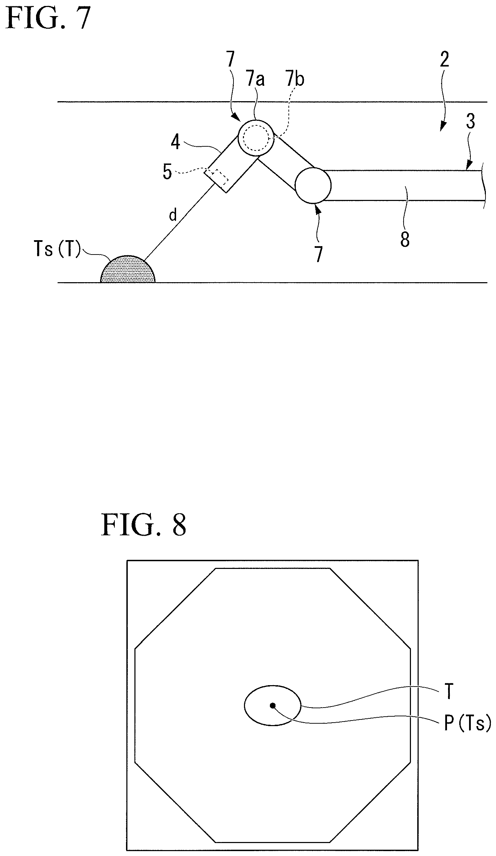

Hereinafter, a first embodiment of the present invention will be described. FIG. 1 is an overall view of a medical system according to the embodiment. FIG. 2 is a partial cross-sectional view of a manipulator of the medical system. FIG. 3 is a schematic diagram showing an operation input device of the medical system. FIGS. 4 and 5 are block diagrams of a main part of the medical system. FIG. 6 is a flowchart for explaining a control procedure in a controller of the medical system. FIGS. 7 to 10 are diagrams for explaining the operation of the medical system.

As shown in FIG. 1, the medical system 1 of the present embodiment includes a manipulator 2, an operation input device 20 (operation part), and a joint control device 50. Further, the medical system 1 of the present embodiment may include an image processing device 30, a display device 35, and a light source device 40 that are commonly known.

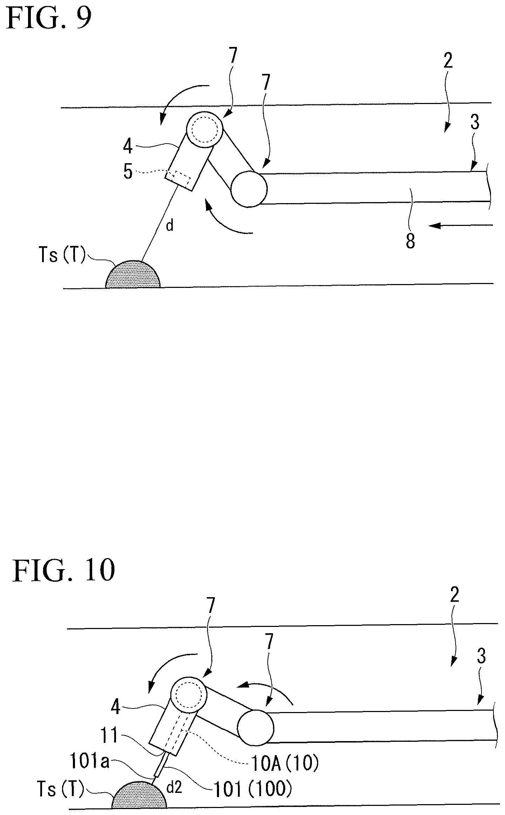

The manipulator 2 shown in FIGS. 1 and 2 is a device which is inserted into a body cavity or a gastrointestinal tract (hereinafter referred to as "in a body or the like") of a patient to observe organs and the like and to treat organs (see FIG. 10). As shown in FIG. 1, the manipulator 2 is connected to the operation input device 20, the joint control device 50, the image processing device 30, the display device 35, and the light source device 40. A commonly known treatment instrument 100 that is capable of being inserted into the flexible endoscope can be attached to the manipulator 2 of the present embodiment. As shown in FIG. 2, the treatment instrument 100 has an elongated insertion body 101 and a treatment instrument type information storage 102 in which the type of the treatment instrument 100 is stored. The treatment instrument type information storage 102 includes, for example, a nonvolatile storage medium readable by the joint control device 50. As the configuration of the treatment instrument 100, for example, a configuration that can be operated electrically and can be remotely controlled by the operation input device 20 may be appropriately adopted. Further, the manipulator 2 is held by the suspension device 110 as necessary.

The manipulator 2 includes an insertion 3, a drive part 15, and a connector 16.

As shown in FIGS. 1 and 2, the insertion 3 is an elongated member which can be used in a state of being inserted into a body or the like. The insertion 3 includes a distal end 4, an elongated part 8, a shape sensor for flexible tube 13, and a proximal end 14. The distal end 4 and the elongated part 8 can be inserted into a body or the like. The distal end 4 is disposed on the distal end side in the insertion direction of the elongated part 8 to be inserted into a body or the like. The proximal end 14 is disposed on the proximal end side of the elongated part 8. A channel 10 for inserting the treatment instrument 100 is disposed inside the insertion 3.

The distal end 4 of the insertion 3 includes a channel distal part 10A, an imager 5, an illumination part 6, and a joint part 7. The distal end 4 is connected to the distal end of the elongated part 8 via the joint part 7.

As shown in FIG. 2, the channel distal part 10A constitutes a part of the channel 10 through which the treatment instrument 100 can be inserted. The channel distal part 10A has a distal opening 11 at the most distal end of the distal end 4 of the insertion part 3. The proximal end of the channel distal part 10A is connected to a channel tube 10B, which will be described later. An insertion body 101 of the treatment instrument 100, which is inserted into the channel 10, can protrude from the distal opening 11 of the channel distal part 10A.

The channel distal part 10A includes: a stopper (not shown) engaged with an insertion body 101 of the treatment instrument 100 inserted into the channel 10; and a switch 10a (insertion-state determination mechanism) which is turned on when the treatment instrument 100 is inserted in the channel 10 and is turned off when the treatment instrument 100 is not inserted in the channel 10.

The stopper defines the upper limit value of the length at which the treatment instrument 100 protrudes from the distal opening 11 of the channel distal part 10A. In the present embodiment, the medical system 1 is used in a state where the stopper and the treatment instrument 100 are engaged. Therefore, in the medical system 1 of the present embodiment, the length of the treatment instrument 100 in use protruding from the distal opening 11 of the channel 10 is known. This length may be different depending on the type of treatment instrument 100. In addition, each treatment instrument 100 may be configured so that the lengths of various treatment instruments 100 protruding from the distal opening 11 of the channel 10 are equal to each other even with different treatment tools 100.

A commonly known endoscopic treatment instrument having a flexible insertion body can be inserted into the channel 10 and used.

The imager 5 is an end effector that acquires an image of a target to be treated. The imager 5 is disposed at a distal portion of the distal end 4. The optical axis of the imager 5 extends from the most distal end of the distal end 4 toward the front of the distal end 4. For example, the optical axis of the imager 5 extends parallel to the longitudinal axis of the distal end 4 of the insertion 3. Thereby, the imager 5 of the present embodiment has an imaging field of view at a front region of the distal end 4 of the insertion 3. As the imager 5, the structure of the observation means in a commonly known endoscope such as a CCD imaging device can be appropriately selected and adopted.

The imager 5 can capture an image for measuring the distance to the object captured in the field of view. For example, the imager 5 includes a set of imaging elements capable of imaging a set of images (a first image and a second image) having parallax. The imager 5 may have an optical system for forming a set of images having parallax on one imaging device, instead of having a set of imaging elements in the imager 5. A set of images captured by the imager 5 is used for distance measurement by stereo measurement in the controller 51, which will be described later. The imager 5 need not have a function of acquiring an image for distance measurement. In this case, a commonly known distance measuring mechanism such as a laser distance measuring device is arranged at the distal end 4 of the insertion 3.

The imager 5 outputs a signal (image signal) of a set of images that has been acquired to the image processing device 30 via the connector 16, which will be described later.

The illumination part 6 illuminates the imaging field of view of the imager 5 using the light transmitted from the light source device 40 through an optical fiber (not shown). In the case where the light source device 40 is not provided, the illumination part 6 may have a light emitting element such as an LED that emits illumination light toward the front of the distal end 4.

The joint part 7 includes a plurality of joints 7a having a rotation axis, a plurality of encoders 7b provided corresponding to the respective joints 7a, and an arm 7c connecting two adjacent joints 7a.

The joint 7a is arranged at both ends of the arm 7c. Thereby, the joint part 7 can be deformed so as to be bent at the rotation axis of the joint 7a. The encoder 7b of the joint part 7 can detect the amount of operation of the corresponding joint 7a. The joint part 7 of the present embodiment has a plurality of encoders 7b, which individually detect the amount of operation of the corresponding joint 7a, on the joint 7a. The encoder 7b may be provided in the motor of the drive part 15 for operating the joint 7a. In the case where the encoder 7b is disposed on the motor of the drive part 15, the encoder 7b detects the amount of drive of the motor of the drive part 15. In this case, the controller 51 can calculate the amount of operation of the joint 7a based on the information indicating the relationship between the amount of drive of the motor and the amount of operation of the joint 7a.

The joint part 7 has a number of degrees of freedom corresponding to the number of the joints 7a. Although FIGS. 1 to 3 show examples in which two joints 7a are provided, the number of joints 7a is not limited to two. Further, each joint 7a may correspond to each degree of freedom of roll, pitch and yaw. The joint 7a is not limited to a rotating joint, and may be a one that moves forward and backward.

The elongate part 8 has a flexible tube 9, the channel tube 10B, and a channel proximal part 10C. An angle wire for connecting the joint part 7 and the drive part 15 is inserted into the elongated part 8. In the present embodiment, a plurality of angle wires corresponding to the respective joints 7a are inserted into the elongated part 8 so that the joint 7a of the joint part 7 cab be operated individually.

The flexible tube 9 is a flexible tubular member connecting the distal end 4 and the proximal end 14 of the insertion 3. The distal end of the flexible tube 9 is connected to the proximal end of the joint part 7. The flexible tube 9 communicates with the distal end 4 and the proximal end 14 of the insertion 3.

The channel tube 10B is a flexible tubular member disposed inside the flexible tube 9. The channel tube 10B constitutes a part of the channel 10 through which the treatment instrument 100 can be inserted. The distal end of the channel tube 10B is connected to the proximal end of the channel distal part 10A. The proximal end of the channel tube 10B is connected to the distal end of the channel proximal part 10C.

The channel proximal portion 10C constitutes a part of the channel 10 through which the treatment instrument 100 can be inserted. The channel proximal part 10C has a proximal opening 12. The insertion body 101 of the treatment instrument 100 can be inserted from the proximal opening 12 of the channel proximal part 10C.

The shape sensor for flexible tube 13 has magnetic sensors at a plurality of locations of the flexible tube 9 in order to detect the shape of the flexible tube 9 by the joint control device 50. For example, the shape sensor for flexible tube 13 can output information (flexible tube shape information) for calculating the shape of the flexible tube 9. Further, the shape sensor for flexible tube 13 is connected to the joint control device 50.

The driving part 15 is connected to the proximal end 14 of the insertion 3. The driving part 15 includes a plurality of motors (actuators) provided corresponding to the respective joints 7a so as to generate motive power for operating the respective joints 7a arranged in the joint part 7, and a pulley that transmits the motive power generated by the motors to the angle wire. The power generated by the drive part 15 is transmitted to the joint part 7 via the above-mentioned angle wire.

The connector 16 is connected to the driving part 15. The connector 16 electrically connects the manipulator 2 to the operation input device 20, the image processing device 30, and the joint control device 50.

The connector 16 has a signal line for outputting a signal of an image (image signal) captured by the imager 5 to the image processing device 30, a signal line for outputting the drive signal to the drive part 15 of the manipulator 2 from a controller 51 which will be described later, and a signal line for outputting angle information detected by the encoder 7b of the manipulator 2 to the controller 51 which will be described later.

Further, the connector 16 has an optical fiber that transmits illumination light from the light source device 40 to the manipulator 2.

As shown in FIG. 3, the operation input device 20 includes a master arm 21, a drive part 24, and a mode selector 25.

The master arm 21 has an input part 22, a plurality of master joints 23a, and a plurality of encoders 23b corresponding to each master joint 23a. When the input part 22 of the master arm 21 is moved by the operator, the orientation of the master joint 23a changes.

The encoder 23b is disposed on the corresponding master joint 23a. The encoder 23b detects the amount of operation of the corresponding master joint 23a and outputs the angle information of the master joint 23a to the joint control device 50. The master joint 23a provided at the master arm 21 has a correspondence relationship with the joint 7a provided at the joint part 7 of the manipulator 2. For example, the correspondence relationship database showing the correspondence relationship between the pair of the master joint 23a and the encoder 23b provided at the master arm 21 and the pair of the joint 7a and the encoder 7b provided at the joint part 7 of the manipulator 2 is stored in the controller 51 in the storage 70. When the master joint 23a operates according to the operation of the master arm 21 by the operator, the encoder 23b corresponding to the operated master joint 23a detects the amount of operation of the master joint 23a and outputs the detected amount of operation to the controller 51. The controller 51 specifies the joint 7a corresponding to the encoder 23b to which the amount of operation has been output based on the correspondence relationship database described above. The controller 51 generates a drive signal for operating the specified joint 7a based on the amount of operation detected by the encoder 23b and outputs the generated drive signal to the drive part 15.

The drive part 24 of the operation input device 20 is connected to the joint control device 50 and the master joint 23a. The drive part 24 of the operation input device 20 can operate the master joint 23a according to the drive signal output from the joint control device 50.

The mode selector 25 includes a switch 26 for switching the operation mode of the medical system 1, and a distance input mechanism 27 for manually setting the distance between the imager and the target. The switch 26 and the distance input mechanism 27 are connected to the joint control device 50. The distance input mechanism 27 is, for example, a switch for re-executing setting of the control target value of the distance between the imager 5 and the target T.

The mode selector 25 outputs the mode information to the controller 51 according to the operation by the operator. Examples of the mode information are such as information for designating the operation mode of the medical system 1 and information for re-executing the setting of the control target value.

The image processing device 30 receives an input of an image signal output from the imager 5. Among a pair of images (a first image and a second image) included in the image signal, the image processing device 30 converts a predetermined one of the pair of images (for example, in the present embodiment, the first image) into a video signal of a format suitable for display on the display device 35, based on the image signal output from the imager 5. The image processing device 30 outputs the video signal to the display device 35.

Further, based on the image signal output from the imager 5, the image processing device 30 encodes a signal of the first image included in the image signal into first image data, and encodes a signal of the second image included in the image signal into second image data. The image processing device 30 outputs a set of image data including the first image data and the second image data to the controller 51.

The display device 35 receives input of the video signal output from the image processing device 30. The display device 35 has, for example, a liquid crystal monitor 36.

The joint control device 50 shown in FIGS. 1 and 4 includes a controller 51 that controls the drive part 15 of the manipulator 2 and the drive part 24 of the operation input device 20, and a storage 70 that stores data generated by the controller 51.

As shown in FIGS. 4 and 5, the controller 51 includes an image data receptor 52, a characteristic point setting part 53, a distance measurement part 54, a control target value setting part 55, a distance comparator 56, a determinator 57, an orientation calculator 58, a characteristic point extraction part 59, a characteristic point position calculator 60, an orientation-deviation calculator 61, a distance-deviation calculator 62, a correction amount calculator 63, an operation command generator 64, a drive signal generator 65, and an insertion-state detector 66.

The image data receptor 52 receives input of a set of image data output from the image processing device 30. The image data receptor 52 outputs a set of image data to the characteristic point setting part 53. The image data receptor 52 outputs the first image data of the set of image data to the characteristic point extraction part 59 and the distance measurement part 54.

The characteristic point setting part 53 receives input of a set of image data output from the image data receptor 52. The characteristic point setting part 53 extracts a plurality of characteristic points based on shape, color, or the like at a characteristic point setting timing which will be described later, based on the first image data of the set of image data, with respect to a predetermined range including the center of the first image. The center of the first image in the present embodiment corresponds to the target position Ts in the lock-on mode, which will be described later. The characteristic point setting part 53 outputs the information including the characteristic point data indicating the extracted characteristic point and the center point data indicating the center of the first image to the storage 70 as a feature point pattern. Further, the characteristic point setting part 53 outputs the characteristic point pattern to the distance measurement part 54.

The distance measurement part 54 receives input of a set of image data output from the image data receptor 52. Also, the distance measurement part 54 reads the characteristic point pattern stored in the storage 70. The distance measurement part 54 extracts an area corresponding to the characteristic point pattern in the second image data of the set of image data using a method of characteristic point matching. The distance measurement part 54 converts the amount of displacement of the region corresponding to the characteristic point pattern in the first image data and the second image data into the distance value to the portion where the characteristic point pattern is set. In the above conversion by the distance measurement part 54, the distance measurement part 54 acquires a distance value from a reference point predefined in the imager 5 to a portion where the characteristic point is set. The distance measurement part 54 outputs the acquired distance value to the control target value setting part 55 and the distance comparator 56.

The control target value setting part 55 receives input of the distance value output from the distance measurement part 54. The control target value setting part 55 outputs the distance value as the control target value to the storage 70 after the first distance measurement in the lock-on mode which will be described later and after the distance measurement based on input to the distance input mechanism 27.

The distance comparator 56 receives input of the distance value output from the distance measurement part 54. Further, the distance comparator 56 reads the control target value stored in the storage 70.

The distance comparator 56 compares the distance value output from the distance measurement part 54 with the value of (control target value - threshold value). For example, the distance comparator 56 outputs the value of {distance value - (control target value - threshold value)} to the determinator 57 as a comparison result.

Further, the distance comparator 56 compares the distance value output from the distance measurement part 54 with the value of (control target value + threshold value). For example, the distance comparator 56 outputs the value of {distance value - (control target value + threshold value)} to the determinator 57 as a comparison result.

The threshold value is a predetermined value based on the distance that is allowable as an error with respect to the control target value. The absolute values of the respective threshold values may be equal to each other or different from each other.

When the control target value is not stored in the storage 70, the distance comparator 56 does not operate.

The determinator 57 receives input of the comparison result output from the distance comparator 56. Based on the comparison result, the determinator 57 determines whether or not to output the calculation start command and the difference information to the distance-deviation calculator 62, and whether to output the distance correction start command to the correction amount calculator 63.

As an example, when determining that the distance value is equal to or less than the value of (control target value - threshold) or equal to or more than the value of (control target value + threshold), the determinator 57 outputs the calculation start command and the difference information to the distance-deviation calculator 62. The operation start command is a command for causing the distance-deviation calculator 62 to start an operation for calculating an amount of distance-deviation, which will be described later. The difference information is information indicating the difference between the current distance value measured by the distance measurement part 54 and the control target value.

Further, in this case, the determinator 57 outputs a distance correction start command to the correction amount calculator 63. When the distance value exceeds the value of (control target value - threshold value) and is less than the value of (control target value + threshold), the determinator 57 does not output command or the like to the distance-deviation calculator 62 and command to the correction amount calculator 63.

The orientation calculator 58 receives input of flexible tube-shape information output from the shape sensor for flexible tube 13 of the manipulator 2. Further, the orientation calculator 58 receives input of angle information output from the encoder 7b of the manipulator 2. The orientation calculator 58 calculates the orientation of the flexible tube 9 of the manipulator 2 based on the flexible tube-shape information. Further, the orientation calculator 58 calculates the orientation of the joint part 7 based on the angle information from the encoder 7b. The orientation calculator 58 calculates information (orientation information) indicating the orientation of the imager 5 positioned at the distal end of the joint part 7, based on the calculation results of the orientation of the flexible tube 9 and the joint part 7. The orientation information calculated by the orientation calculator 58 includes, for example, data such as coordinates indicating the orientation of the flexible tube 9 and the joint part 7 in a three-dimensional orthogonal coordinate system (hereinafter referred to as "reference coordinate system") whose origin is the center of the flexible tube 9 at the boundary between the flexible tube 9 and the joint part. The orientation information includes data such as coordinates indicating the orientation of the imager 5 disposed at the distal end of the joint part 7.

The orientation calculator 58 outputs the orientation information of the imager 5 to the characteristic point position calculator 60, the orientation-deviation calculator 61, and the distance-deviation calculator 62.

The characteristic point extraction part 59 receives input of the first image data output from the image data receptor 52. Further, the characteristic point extraction part 59 reads the characteristic point pattern from the storage 70.

The characteristic point extraction part 59 performs characteristic point mapping on the first image data using the characteristic point pattern. As a result, the characteristic point extraction part 59 can extract a region corresponding to the characteristic point in the first image based on the first image data. The extraction result by the characteristic point extraction part 59 includes, for example, information on the position of the point (the current position of the target position) corresponding to the central point data included in the characteristic point pattern on the first image.

The characteristic point extraction part 59 outputs the extraction result of the characteristic point to the characteristic point position calculator 60.

The characteristic point position calculator 60 receives input of the orientation information output from the orientation calculator 58. Furthermore, the characteristic point position calculator 60 receives input of the extraction result output from the characteristic point extraction part 59. Based on the orientation information and the extraction result, the characteristic point position calculator 60 calculates information (positional relationship information) indicating the current position of the target position relative to the imager 5.

The characteristic point position calculator 60 outputs the positional relationship information to the orientation-deviation calculator 61 and the distance-deviation calculator 62.

The orientation-deviation calculator 61 receives input of the orientation information output from the orientation calculator 58. Further, the orientation-deviation calculator 61 receives input of the positional relationship information output from the characteristic point position calculator 60.

The orientation-deviation calculator 61 calculates the amount of orientation deviation of the imager 5 in the reference coordinate system based on the orientation information and the positional relationship information.

The orientation-deviation calculator 61 outputs the calculated amount of orientation deviation to the correction amount calculator 63.

The distance-deviation calculator 62 receives input of the orientation information output from the orientation calculator 58. Further, the distance-deviation calculator 62 receives input of the positional relationship information output from the characteristic point position calculator 60. The distance-deviation calculator 62 reads the control target value stored in the storage 70.

The distance-deviation calculator 62 starts calculation of the amount of distance deviation in accordance with the operation start command output from the determinator 57. First, based on the orientation information and the positional relationship information, the distance-deviation calculator 62 sets the direction of the straight line, which connects the current position of the target position relative to the imager 5 with the imager 5, as the moving direction of the imager 5. Next, the distance-deviation calculator 62 sets the distance difference, which is output from the determinator 57 as an argument of the operation start command, as the amount of advance / retreat of the imager 5 in the moving direction of the imager 5. The distance-deviation calculator 62 outputs information including the moving direction and the amount of advance / retreat of the imager 5 to the correction amount calculator 63 as the amount of distance deviation.

The correction amount calculator 63 receives input of the amount of orientation deviation output from the orientation-deviation calculator 61. Further, the correction amount calculator 63 receives input of a distance correction start command output from the determinator 57. The correction amount calculator 63 can receive input of the amount of distance deviation in a state in which the distance correction start command is input.

Based on the amount of orientation deviation, the correction amount calculator 63 generates a correction command (orientation correction command) for operating each joint 7a of the joint part 7. As an example, the correction amount calculator 63 specifies the joint 7a to be operated, calculates angle information indicating the amount of operation of the specified joint 7a based on the amount of orientation deviation, and generates a correction command.

Further, the correction amount calculator 63 generates a correction command (distance correction command) for operating each joint 7a of the joint part 7 based on the amount of distance deviation. As an example, the correction amount calculator 63 specifies the joint 7a to be operated, calculates angle information indicating the amount of operation of the specified joint 7a based on the amount of distance deviation, and generates a correction command. The distance correction command is generated when there is input of a distance correction start command.

If a distance correction start command is input, the correction amount calculator 63 generates a distance correction command as a correction command, and if there is no input of a distance correction start command, the correction amount calculator 63 generates an orientation correction command as a correction command based on input of the amount of orientation deviation. When a distance correction start command is input, the correction amount calculator 63 generates a distance correction command as a correction command, and if there is no input of the distance correction start command, the correction amount calculator 63 calculates a posture Thereby generating a correction command. When the distance correction start command is not input after the generation of the orientation correction command, the correction amount calculator 63 outputs the orientation correction command to the operation command generator 64. When there is an input of the distance correction start command, the correction amount calculator 63 outputs the orientation correction command and the distance correction command to the operation command generator 64 after generating the orientation correction command and the distance correction command.

The operation command generator 64 receives input of the correction command output from the correction amount calculator 63. Further, the operation command generator 64 receives input of the angle information output from the encoder 23b of the operation input device 20. Further, the operation command generator 64 receives input of the mode information output from the mode selector 25 of the operation input device 20.

The operation command generator 64 selects either the manual mode or the lock-on mode according to the mode information. The operation command generator 64 operating in the manual mode generates an operation command based on the angle information output from the encoder 23b of the operation input device 20 and outputs the generated operation command to the drive signal generator 65. The operation command generator 64 operating in the lock-on mode generates an operation command based on the angle information output from the encoder 23b of the operation input device 20 and the angle information based on the correction command, and outputs the generated operation command to the drive signal generator 65.

The drive signal generator 65 receives input of the operation command output from the operation command generator 64. The drive signal generator 65 generates a drive signal for driving the drive part 15 of the manipulator 2 based on the operation command. The drive signal generator 65 generates a drive signal for driving the drive part 24 of the operation input device 20 so that the master arm 21 maintains a similar shape to the joint part 7.

As shown in FIG. 5, the insertion state detector 66 receives input of an ON / OFF signal output from the switch 10a.

When an ON signal is input from the switch 10a, the insertion state detector 66 reads information (type information) specifying the type of the treatment instrument 100 from the treatment instrument type information storage 102. In the present embodiment, based on the type information read by the insertion state detector 66 from the treatment instrument type information storage 102, the insertion state detector 66 calculates or acquires the length by which the treatment instrument 100 protrudes from the distal opening 11 of the channel 10. For example, the length by which the treatment instrument 100 protrudes from the distal opening 11 of the channel 10 is stored in advance in the storage 70 as a database in a state of being associated with the type information of the treatment instrument 100, and the insertion state detector 66 reads the length by which the treatment instrument 100 protrudes from the distal opening 11 of the channel 10 from the storage 70 based on the type information. The insertion state detector 66 sets the length, which is read based on the type information, as a new control target value (predetermined distance d2), and outputs a command (update command) for updating the control target value by the predetermined distance d2 to the control target value setting part 55, using the predetermined distance d2 as an argument.

When an OFF signal is input from the switch 10a, the insertion state detector 66 outputs a command for updating the control target value to the control target value setting part 55 so that the control target value again becomes a value based on the distance value.

The controller 51 may be configured to be capable of acquiring information such as the insertion amount of the insertion 3 from a trocar or a mouthpiece that can measure the insertion amount of the insertion 3 into the body. In this case, the controller 51 can use the result of measuring advancement / retraction and rotation of the insertion 3 in the body using the controller 51 connected to the trocar or the mouthpiece, to calculate the change in the position and orientation of the imager 5.

The operation of the controller 51 will be described.

The controller 51 has two operation modes, a manual mode (normal mode) and a lock-on mode, as operation modes for operating the drive part 15 of the manipulator 2.

In the manual mode, the controller 51 generates a drive signal based on the operation of the master arm 21 by the user, and transmits the drive signal to the drive part 15 of the manipulator 2. As a result, the joint part 7 of the manipulator 2 assumes an orientation that follows the shape of the master arm 21.

The lock-on mode is a mode for automatically controlling the operation of the joint part 7 so as to continuously capture the target at the center of the imaging field of view of the imager 5. The lock-on mode is started in accordance with an operation in which the operator sets the operation mode to the lock-on mode using the switch 26 of the mode selector 25.

In the lock-on mode, the drive signal generator 65 automatically generates the drive signal so that the position (the target position Ts) of the target object to be imaged by the imager 5 is always located at the center of the field of view of the imager 5, and transmits the generated drive signal to the driving part 15 of the manipulator 2. Therefore, for example, when the user moves the insertion 3, the operation of the joint part 7 is controlled by the controller 51 so that the imager 5 faces the target position Ts even after the user moves the insertion 3. That is, during the lock-on mode, the drive part 15 is automatically driven by the controller 51 so that the imager 5 faces the target position Ts no matter how the user moves the manipulator 2. During the lock-on mode, the controller 51 automatically controls the position and orientation of the imager 5 so that the target position Ts is located at the center of the field of view of the imager 5. As a result, during the lock-on mode, the target position Ts is always located at the center of the field of view image displayed on the monitor 36 (see FIG. 1) of the display device 35.

Further, during the lock-on mode, the controller 51 can set the target position Ts again according to the input by the operator for the distance input mechanism 27 electrically connected to the controller 51. For example, the controller 51 acquires the image captured by the imager 5 from the image processing device 30, and recognizes the center P of the field of view (see FIG. 8) of the imager 5 at the time when input to the distance input mechanism 27 is changed as the target position Ts. In this case, the time point when there is an input by the operator on the distance input mechanism 27 is the above-described characteristic point setting timing and the above-described distance setting timing.

At this time, the controller 51 can recognize the target position Ts by recognizing the characteristic point of the center of the field of view (the target position Ts) of the imager 5 even when the target position Ts moves from the center of the field of view. The characteristic point can be acquired from the image by a commonly known characteristic point recognition technology such as SIFT (Scale-invariant feature transform) or SURF (Speed-Upped Robust Feature), based on, for example, the surface shape of internal organs in the body, vascular traveling in the image acquired by the imager 5 by narrow-band light observation, coloring state by the pigment dispersed in the organs, marking attached to the organ surface by cauterization of organs, or the like.

Further, during the lock-on mode, the joint part 7 of the manipulator 2 is automatically controlled by the controller 51. At this time, the movable range of the master joint 23a is limited by the controller 51 so that the orientation of the joint part 7 of the manipulator 2 changed by automatic control is reflected (limiting step). For example, the controller 51 has a function of transmitting a drive signal for operating the master arm 21 in response to automatic control of the joint part 7 of the manipulator 2 by the controller 51 during the lock-on mode to the drive part 24 of the operation input device 20. As a result, the similarity relationship between the joint part 7 of the manipulator 2 and the master arm 21 is maintained during the lock-on mode. The master arm 21 in the lock-on mode can be operated by the operator within a range where the similarity relationship between the joint part 7 and the master arm 21 is maintained. Therefore, the joint part 7 of the manipulator 2 is automatically controlled by the controller 51 so that the operator can change the viewpoint position during the lock-on mode and the imager 5 is directed to the target position Ts even after changing the viewpoint position.

The operation method of the medical system 1 of the present embodiment will be described together with the operation of the medical system 1.

### (Observation of target to be treated)

First, a method of operating the medical system 1 in the case of observing a treatment target using the medical system 1 of the present embodiment will be described.

When the medical system 1 is activated, the medical system 1 operates in the manual mode. That is, the initial state of the switch 26 in the mode selector 25 is such that the controller 51 is set to the manual mode. When the lock-on mode is not set by the input to the switch 26 (No, in step S1), the controller 51 continues to operate in the manual mode (step S13).

As shown in FIG. 8, in the manual mode, the operator actuates the distal end 4 of the manipulator 2 by using the master arm 21 so that the desired part (target) as the treatment target is displayed on the image displayed on the monitor 36 of the display device 35.

Then, when the target T is positioned in the vicinity of the center of the image displayed on the monitor 36 of the display device 35 (see FIG. 8), the operator operates the switch 26 of the mode selector 25 to start the lock-on mode.

When the operator operates the switch 26 to start the lock-on mode, the operation mode of the controller 51 (the operation mode of the entire of the medical system 1) is switched to the lock-on mode based on the mode information output from the mode selector 25 (Yes, in step S1).

When the operation mode of the controller 51 is set to the lock-on mode, the controller 51 starts the operation in the lock-on mode (step S2).

When the controller 51 starts operation in the lock-on mode, the characteristic point setting part 53 acquires the characteristic point of the object located at the position of the center P of the field of view of the imager 5 (the position on the extended line of the optical axis of the imager 5, see FIG. 8), recognizes a portion having this characteristic point as a target object to be tracked by the imager 5, and sets the recognized portion as the target point Ts (characteristic point recognition step, step S3).

Subsequently, the distance measurement part 54 calculates the distance between the target position Ts and the imager 5 (distance measuring step, step S4).

Subsequently, based on the distance measured by the distance measurement part 54, the control target value setting part 55 sets a predetermined distance d to be the control target value (step S5).

The above distance measured first by the distance measurement part 54 of the controller 51 after the operation in the lock-on mode is started is stored in the storage 70 as a control target value (predetermined distance d) maintained in the lock-on mode. Further, the operator can directly input the predetermined distance d with a numerical value or the like, as necessary. In this case, the predetermined distance d stored in the controller 51 is the value input by the operator.

In a state in which the medical system 1 is operating in the lock-on mode, the operator can perform an input for operating the joint part 7 of the manipulator 2 by operating the master arm 21 of the operation input device 20 as necessary.

When the operator operates the master arm 21, angle information is output from the encoder 23b provided on the master arm 21 to the operation command generator 64. Further, the controller 51 operating in the lock-on mode repeatedly determines whether or not the target is located within a predetermined range around the optical axis in the orientation-deviation calculator 61. When the orientation-deviation calculator 61 determines that the target is not within the predetermined range around the optical axis, the controller 51 controls the correction amount calculator 63 to generate a correction command for operating the drive part 15 so that the position of the target on the image is closer to the center of the image (position of the optical line of the imager 5). The correction amount calculator 63 outputs the correction command to the operation command generator 64.

Based on the angle information output from the encoder 23b of the master arm 21 to the operation command generator 64 and the correction command output from the correction amount calculator 63 to the operation command generator 64, the operation command generator 64 generates an operation command for actuating the joint part 7 (step S6). The operation command generator 64 outputs the operation command to the drive signal generator 65.

Subsequently, the drive signal generator 65 generates a drive signal based on the operation command and outputs the drive signal to the drive part 15 that operates the joint part 7 of the manipulator 2 (step S7).

Subsequently, the controller 51 calculates a distance value between the target position Ts and the imager 5 (step S8). The controller 51 controls the distance measurement part 54 to calculate the distance between the target (target position Ts) and the imager 5 at a predetermined interval.

Subsequently, the controller 51 determines whether or not the distance between the target position Ts and the imager 5 exceeds the value of (control target value - threshold value) and whether or not the distance between the target position Ts and the imager 5 is less than the value of (control target value + threshold value) (step S9). The controller 51 of the present embodiment controls the distance comparator 56 to compare the distance value output from the distance measurement part 54 with the value of (control target value - threshold value), and to compare the distance value with the value of (control target value + threshold value). Furthermore, the controller 51 of the present embodiment controls the determinator 57 to determine whether or not the distance value between the target position and the imager exceeds the value of (control target value - threshold value) and is less than the value of (control target value + threshold value) (step S9).

In step S9, when the determinator 57 determines that the distance value between the target position Ts and the imager 5 exceeds the value of (control target value - threshold value) and is less than the value of (control target value + threshold), the distance between the target position Ts and the imager 5 is a distance having an error whose range is equal to or less than the threshold value with respect to the control target value. Therefore, the controller 51 skips the control (steps S10 and S11) of the distance between the target position Ts and the imager 5 (Yes, in step S9).

When the determinator 57 determines that the distance value between the target position Ts and the imager 5 is equal to or less than the value of (control target value - threshold value) or equal to or more than the value of (control target value + threshold value) in step S9, the distance between the target position Ts and the imager 5 is a distance having an error exceeding the threshold value with respect to the control target value. Therefore, the controller 51 controls to proceed to step S10 so as to control the distance between the target position Ts and the imager 5 (No, in step S9).

In step S10, the controller 51 calculates the amount of operation of the drive part 15 for moving the imager 5 so that the distance value between the target position Ts and the imager 5 is the control target value (correction amount calculation step). The controller 51 according to the present embodiment controls the distance-deviation calculator 62 to calculate the amount of distance deviation, and subsequently controls the correction amount calculator 63 to generate a correction command including the amount of operation (angle information) of each joint 7a of the joint part 7 to output the generated correction command to the operation command generator 64. The operation command generator 64 generates an operation command based on the correction command (distance correction command) output from the correction amount calculator 63, and outputs the generated operation command to the drive signal generator 65.

Subsequently, the drive signal generator 65 generates a drive signal based on the operation command and outputs the drive signal to the drive part 15 of the manipulator 2 (step S11).

After completion of step S11, the controller 51 refers to the current operation mode of the controller 51 and determines whether or not the operation mode is the lock-on mode (step S12). If it is determined in step S12 that the operation mode is the lock-on mode (Yes, in step S 12), the process proceeds to step S6 described above. While the operation of the controller 51 in the lock-on mode is continued, each step from step S6 to step S12 is repeated. On the other hand, if it is determined in step S12 that the operation mode is not the lock-on mode (No, in step S12), the process proceeds to step S1.

As shown in FIG. 7 and FIG. 9, by each step from step S1 to step S12, even if the positional relationship between the manipulator 2 and the target T (target position Ts) changes, the controller 51 can move the joint part 7 so that target T is located within a predetermined range at the center of the image and the distance between the target T and the imager 5 is maintained to be the predetermined distance d.

As described above, in the present embodiment, when the lock-on mode is set while the target is being observed, the controller 51 controls the operation of the joint part 7 so that the imager 5 tracks the target corresponding to the relative movement between the target T and the manipulator 2, thereby, it is possible to continue observing the target at a viewpoint and a distance suitable for observation.

### (Treatment for target)

Next, a method of operating the medical system 1 in the case where a target is treated in a state in which the target is captured in the field of view of the imager 5 will be described with reference to FIGS. 1, 2 and 10.

When performing a treatment on the target, the operator inserts the desired treatment instrument 100 into the channel 10 as shown in FIG. 10. The treatment instrument 100 is used with the distal end of the treatment instrument 100 protruding from the distal opening 11 of the channel 10.

When a treatment is performed on the target using the treatment instrument 100, a treatment part 101a (see FIG. 10) arranged at the distal end of the treatment instrument 100 needs to reach the position of the target. In the present embodiment, the length by which the treatment instrument 100 inserted through the channel 10 protrudes from the distal opening 11 of the channel 10 is constant as the stopper disposed in the channel 10 engages with the treatment instrument 100. The length of the protrusion of the treatment instrument 100 from the distal opening 11 may differ depending on the type of the treatment instrument 100, but in the present embodiment, based on the type information that the insertion state detection part 66 reads from the treatment instrument type information storage 102, the insertion state detection part 66 acquires the length by which the treatment instrument 100 protrudes from the distal opening 11 of the channel 10. Therefore, the length by which the treatment instrument 100 protrudes from the distal opening 11 of the channel 10 is known. When the treatment instrument 100 is inserted through the channel 10, the controller 51 sets a predetermined distance based on the length by which the treatment instrument 100 protrudes from the distal opening 11 of the channel 10 as the control target value (predetermined distance d2) maintained in the lock-on mode.

The range of the predetermined distance d suitable for observation is determined by the characteristics of the optical system of the imager 5. The range of the predetermined distance d2 suitable for treatment is determined by the configuration of the treatment instrument 100.

In this embodiment, both the imager 5 and the distal opening 11 of the channel 10 are located at the distal end of the distal end 4 so that the imager 5 and the distal opening 11 of the channel 10 are substantially at the same position in the direction of the optical axis of the imager 5. Therefore, by setting the distance between the imager 5 and the target as the predetermined distance d2, the distance from the distal opening 11 of the channel 10 to the target is also substantially equal to the predetermined distance d2. Therefore, the positional relationship between the target and the treatment instrument 100 is maintained in a positional relationship in which treatment can be suitably performed on the target.

As described above, according to the medical system 1 of the present embodiment, the distance between the distal end 4 of the insertion 3 of the manipulator 2 and the target is kept constant, and the state in which the manipulator 2 is directed to the target can be maintained.

In the present embodiment, the controller 51 controls the operation of the drive part 15 so that the target is positioned at the center of the image captured by the imager 5 and the distance between the target and the imager 5 is constant. Therefore, according to the medical system 1 of the present embodiment, it is possible to maintain the distance between the target and the imager 5 to be a constant distance suitable for observing the target, while keeping the target in the center of the field of view of the imager 5.

Furthermore, in the medical system 1 of the present embodiment, the distance between the distal end 4 of the insertion 3 and the target is automatically controlled by the controller 51 to be a distance suitable for treatment of the target using the treatment instrument 100.

### (Second Embodiment)

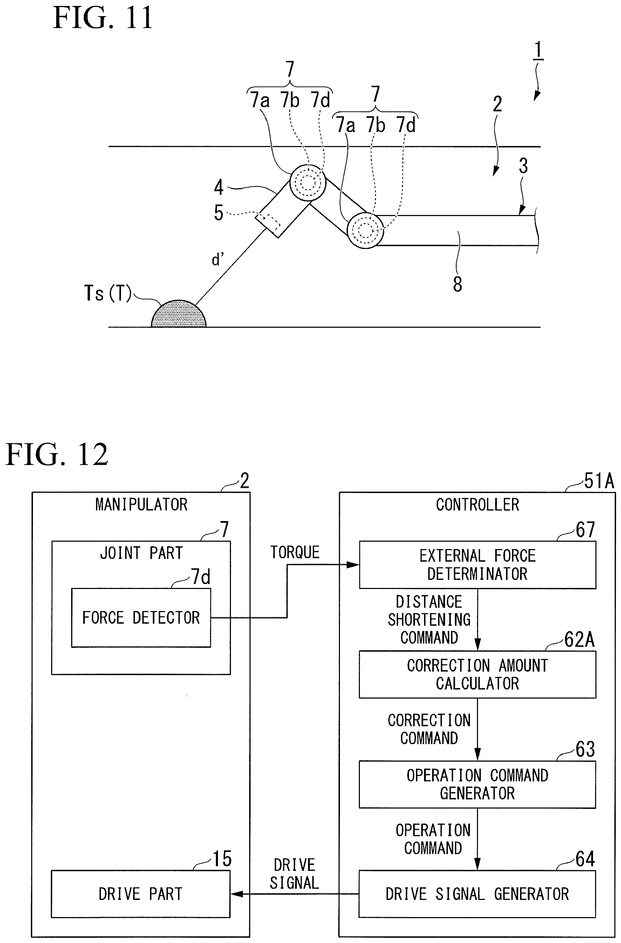

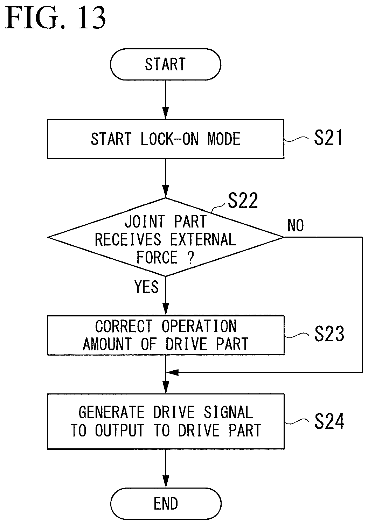

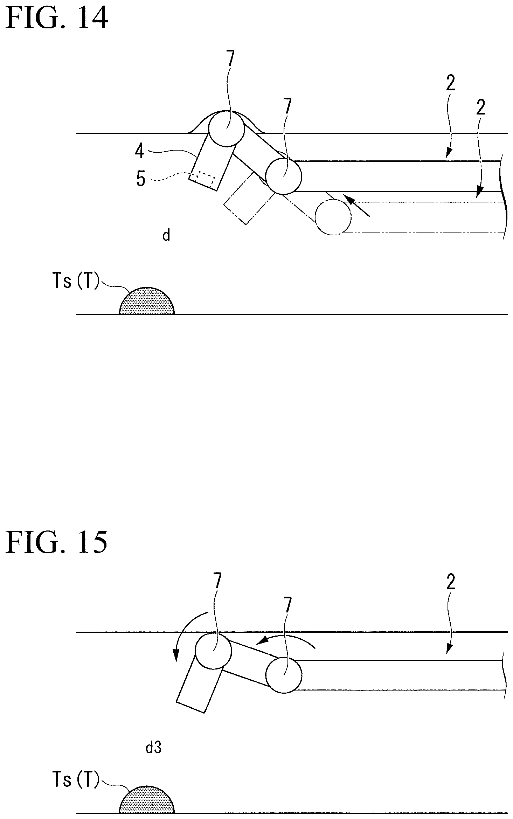

A second embodiment of the present invention will be described. FIG. 11 is an overall view of a medical system according to the present embodiment. FIG. 12 is a block diagram of a main part of the medical system. FIG. 13 is a flowchart for explaining the control procedure in the controller of the medical system. FIGS. 14 and 15 are diagrams for explaining the operation of the medical system.

As shown in FIG. 11, the manipulator 2 of the medical system 1 of the present embodiment further includes a force detector 7d that detects the force applied to the plurality of joints 7a arranged in the joint part 7 from external. As an example, the force detector 7d includes torque sensors individually corresponding to the plurality of joints 7a arranged in the respective joints 7a. As shown in FIG. 12, the force detector 7d is connected to the controller 51A.

The controller 51A of the medical system 1 of the present embodiment is different from the controller 51 disclosed by the first embodiment in that the controller 51A further has a function of changing the operation of the drive part 15 of the manipulator 2 in accordance with the state of force detection by the force detector 7d.

As shown in FIG. 12, the controller 51A of the present embodiment includes an external force determinator 67 that determines whether or not the amount of the torque detected by the torque sensor of the force detector 7d exceeds a predetermined value, a correction amount calculator 63A having a function of shortening the distance between the imager 5 and the target position Ts when it is determined that the torque exceeds the predetermined value by the external force determinator 67. The correction amount calculator 63A is similar to the correction amount calculator 63 of the first embodiment except that the correction amount calculator 63A has the function of shortening the distance between the imager 5 and the target position Ts.

After the start of the lock-on mode (step S21 shown in FIG. 13), the controller 51A of the present embodiment controls the force detector 7d to detect the amount of the external force received by the joint part 7 and output the amount of the external force (torque value in the present embodiment) to the external force determinator 67. Based on the amount of the external force (torque value) output from the force detector 7d, the external force determinator 67 determines whether or not the joint part 7 receives an external force that exceeds a predetermined value (step S22).

If it is determined in step S22 that the joint part 7 receives the external force exceeding the predetermined value, the process proceeds to step S23, and the amount of operation of the drive part 15 of the manipulator 2 is corrected so that the distance between the imager 5 and the target position Ts is slightly shorter than the current distance, to calculate a new amount of operation. For example, as shown in FIG. 7, if the distance between the imager 5 and the target position Ts coincides with the distance set as the control target value, when the manipulator 2 moves as shown in FIG. 14, the joint part 7 and the organ come into contact. At this time, the force detector 7d detects that the joint part 7 is receiving an external force exceeding a predetermined value by detecting a torque exceeding a predetermined value by the torque sensor (the force detector 7d).

In a case in which the distance between the imager 5 and the target position Ts coincides with the distance set as the control target value, when the joint part 7 receives an external force exceeding the predetermined value, the correction amount calculator 63A corrects the amount of operation of the drive part by calculating a new amount of operation of the drive part 15 of the manipulator 2 so that the distance between the imager 5 and the target position Ts is slightly shorter than the control target value. The correction amount calculator 63A outputs a correction command including the corrected amount of operation to the operation command generator 64.

The correction command output from the correction amount calculator 63A to the operation command generator 64 is output to the drive signal generator 65. The drive signal generator 65 generates a drive signal and outputs the generated drive signal to the drive part 15.

When the joint part 7 receives an external force exceeding a predetermined value, the controller 51A according to the present embodiment transmits the drive signal to the drive part 15 of the manipulator 2 so that the distance between the imager 5 and the target position Ts is slightly smaller than the current distance (see FIGS. 14 and 15).

While the external force determinator 67 determines that a force exceeding the predetermined value is applied to the joint part 7, the distance between the imager 5 and the target position Ts continues to decrease. When the external force determinator 67 determines that a force exceeding the predetermined value is not applied to the joint part 7, the controller 51 skips step S23 for shortening the distance between the imager 5 and the target position Ts. As a result, when a force exceeding the predetermined value is not applied to the joint part 7, the decrease of the distance between the imager 5 and the target position Ts stops (distance d3, see FIG. 15). When a force exceeding the predetermined value is not applied to the joint part 7, the controller 51 controls to return the distance between the imager 5 and the target position Ts to the distance set as the control target value. Therefore, the orientation of the joint part 7 is maintained by the controller 51 in a state in which the joint part 7 is subjected to a force equal to or slightly exceeding the predetermined value. In a state in which the distance between the imager 5 and the target position Ts is returned to the control target value, a force equal to or less than the predetermined value is applied to the joint part 7. Or, in a state in which the distance between the imager 5 and the target position Ts is returned to the control target value, an external force is not applied to the joint part 7. Further, when trying to separate the imager 5 and the target position Ts compared to the distance set as the control target value, the controller 51 automatically controls the joint part 7 so as to adjust the distance between the imager 5 and the target position Ts to the control target value.

The controller 51 of the present embodiment can suppress a burden applied to the organs or the like by the joint part 7 to a value close to or less than a predetermined value during the automatic control of the joint part 7 in the lock-on mode. It is preferable that this predetermined value is set to a size that does not cause a burden on organs or the like.

The joint part 7 of the manipulator 2 of the present embodiment is located outside the field of view of the imager 5. Therefore, there are cases in which it is difficult for the operator to recognize the contact state between the joint part 7 and an organ or the like using the manipulator 2 of the present embodiment. In the present embodiment, when the joint part 7 contacts with an organ or the like, the joint part 7 can be moved in a direction in which the joint part 7 separates from the organ or the like, by shortening the distance between the imager 5 and the target position Ts. In the present embodiment, the distance between the imager 5 and the target position Ts is automatically controlled to the longest distance within a range in which the external force applied to the joint part 7 does not exceed a predetermined value. As a result, the manipulator 2 of the present embodiment can satisfactorily achieve both securing of a distance suitable for observation and treatment and reduction of burden on organs or the like.

When the medical system 1 of the present embodiment is used in a state where the distance set as the control target value with respect to the target position Ts is maintained, there is a case in which the manipulator 2 is moved or an organ is moved so that the positional relationship between the manipulator 2 and the target position Ts changes or the pressing force when the joint part 7 of the manipulator 2 contacts an organ or the like changes. In these cases, the medical system 1 according to the present embodiment controls the imager 5 and the imager 5 so that the imager 5 automatically adjusts the distance between the imager 5 and the target position Ts in a state in which the imager 5 continues to capture the target position Ts as the center of the field of view, so that the burden on the organ due to the pressing force on the organ is within an allowable range. That is, in the medical system 1 according to the present embodiment, a state in which the distance between the imager 5 and the target position Ts is maintained at the control target value and a state in which the distance between the imager 5 and the target position Ts is made to be close to the control target value within an allowable range can be automatically switched as necessary. Thus, it is possible for the medical system 1 of the present embodiment to favorably carry out observation and treatment on organs or the like that are targets while giving priority to reduction of the burden on organs.

### (Modification Example)

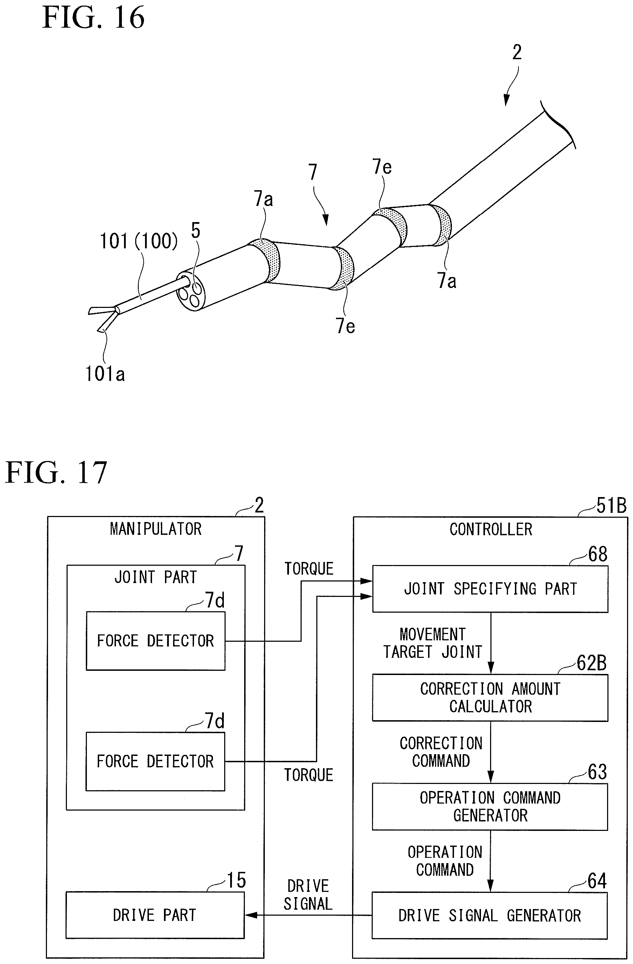

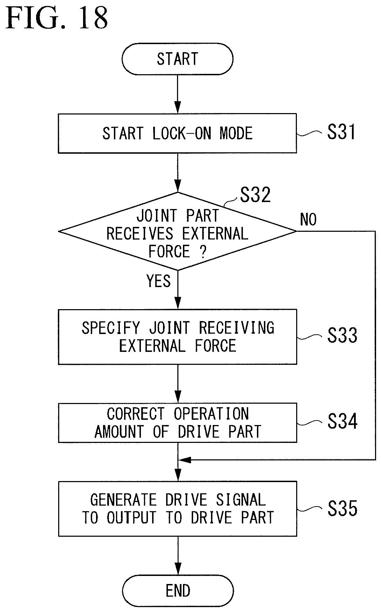

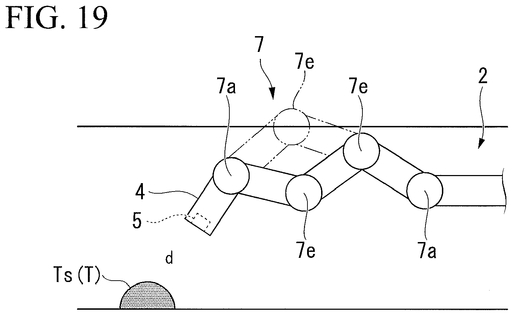

A modification example of the above second embodiment will be described. FIG. 16 is a schematic diagram showing a part of the manipulator of the medical system of the modification example. FIG. 17 is a block diagram showing a main part of the controller of the medical system of the modification example. FIG. 18 is a flowchart showing a control procedure in the controller of the medical system. FIG. 19 is a diagram for explaining the operation of the medical system.

The joint part 7 of the manipulator 2 in the modification example shown in FIG. 16 has redundancy degrees of freedom exceeding degrees of freedom indispensable for controlling the position and orientation of the imager 5. The number of degrees of freedom which is indispensable for the joint part 7 may be determined in consideration of the shape of the part that is the target into which the manipulator 2 is inserted. For example, when the manipulator 2 is inserted into a complicatedly curved hollow organ (for example, small intestine), it is preferable that the number of degrees of freedom for bending the joint part 7 along the shape of the hollow organ is large.

The joint part 7 of the manipulator 2 of the modification example can take various shapes with respect to one position and orientation of the imager 5. For example, the joint part 7 of the manipulator 2 has a redundant joint 7e that is operated only when using redundancy degrees of freedom, in addition to the joint 7a that is commonly used. In the redundant joint 7e of the modification example, the same encoder 7b as that in the first embodiment is disposed. Thereby, when the redundant joint 7e is used, the operation of the common joint 7a and the redundant joint 7e can be acquired by the controller 51 (see FIG. 1).

As shown in FIG. 17, the controller 51 of the modification example includes a joint specifying part 68 that specifies a joint 7a to which an external force exceeding a predetermined value is applied among the plurality of joints 7a.

Further, the controller 51 of the modification example is different from the second embodiment in that, instead of the correction amount calculator 63A disclosed in the second embodiment, a correction amount calculator 63B is provided for calculating a new amount of operation for correcting the drive part 15 so that the force applied to the joint 7a (or the redundant joint 7e) specified by the joint specifying part 68 is equal to or less than a predetermined value and the position and orientation of the imager 5 are maintained.

In the modification example, after the lock-on mode is started (step S31 shown in FIG. 18) in the medical system 1, the joint specifying part 68 shown in FIG. 17 acquires the amount (torque) of force output from a plurality of force detectors 7d (for example, torque sensors), and determines whether or not the joint part 7 receives an external force exceeding a predetermined value (step S32).

In a case where the joint part 7 receives an external force exceeding a predetermined value, the joint specifying part 68 outputs information (movement target joint) that specifies a joint 7a (or a redundant joint 7e) receiving an external force exceeding the predetermined value among the plurality of joints 7a (or the redundant joint 7e) to the correction amount calculator 63B.

The correction amount calculator 63B generates a correction command including the amount of operation for moving the joints 7a and 7e so as to alleviate external forces of the specified joints 7a and 7e as being subjected to an external force exceeding a predetermined value, and outputs the generated correction command to the operation command generator 64.

The operation command generator 64 generates an operation command according to the correction command and outputs the generated operation command to the drive signal generator 65.

The drive signal generator 65 generates a drive signal for moving the joint 7a (or the redundant joint 7e) specified by the joint specifying part 68 according to the operation command, and outputs the generated drive signal to the drive part 15.

In the modification example, as shown in FIG. 19, the controller 51 automatically controls the orientation of the joint part 7 using all the degrees of freedom including the redundancy degree of freedom so as to maintain the position and orientation of the imager 5 while alleviating the external force applied to the joint 7a or 7e to which external force exceeding a predetermined value is applied among a plurality of joints 7a (or redundant joints 7e) provided in the joint part 7. Further, in the modification example, since the joint part 7 has redundancy degree of freedom, the controller 51 automatically can control the orientation of the joint part 7 using all degrees of freedom including redundancy degree of freedom so as to maintain the position and orientation of the imager 5.

When the controller 51 cannot calculate the amount of operation capable of maintaining the position and orientation of the imager 5, the controller 51 decreases the distance between the imager 5 and the target position Ts in the same manner as in the second embodiment.

According to the modification example, since the position and orientation of the imager 5 are maintained as much as possible, it is possible to more stably obtain a state suitable for observation or treatment.

Although the embodiments of the present invention have been described in detail with reference to the drawings, specific configurations are not limited to this embodiment, and design changes and the like within the scope not deviating from the gist of the present invention are included.

For example, an endoscope may be inserted into the channel of the manipulator, not limited to the treatment instrument. When an endoscope is inserted into the channel of the manipulator, the imager of the manipulator and the imager of the endoscope may be used in combination.

In addition, in a state in which the joint part of the manipulator has a plurality of joints, when the controller determines that the external force is applied to all of the joints, the controller may determine the arrangement of each joint so that the amount of the external force applied to all of the joints is within the range between a predetermined upper limit and a predetermined lower limit. In this case, it is possible to reduce the burden on organs and the like by avoiding locally compressing organs or the like by the joint part.

Further, the controller may have a function of detecting the direction of the force which the joint part receives from the external. In this case, based on the direction of the external force applied to the joint part, the controller can easily determine to which side the joint is to be moved in order to ease the external force.

Further, a manipulator having a channel through which an endoscope can be inserted does not need to have an imager. For example, the manipulator has a medical instrument (for example, a forceps, a knife, a clip, a stapler, and the like) for performing a treatment on a treatment target portion as an end effector at a distal end of the insertion, and the endoscope inserted in the channel of a manipulator has an imager for observation with respect to a treatment target portion. In this case, the medical system is configured with the manipulator and the endoscope being combinable. Further, in this case, the joint control device of the medical system can control the medical system in the lock-on mode by operating the joint part of the manipulator based on the image acquired by the endoscope.

Instead of the switch for determining whether or not the treatment instrument is inserted into the channel, the controller may have an image analysis part (not shown) that outputs an on/off signal to the insertion state detector based on whether or not the treatment instrument is positioned within the imaging field of view of the imager.

Also, instead of the force detector including the torque sensor, the force detector may include a strain gauge.

Additionally, the constituent elements illustrated in the above-described respective embodiments and respective modification examples can be suitably configured in combination.

### [Industrial Applicability]

The invention is applicable to a medical system.

### [Reference Signs List]

- 1: medical system
- 2: manipulator
- 3: insertion
- 4: distal end of insertion
- 5: imager
- 6: illumination part
- 7: joint part
- 7a: joint
- 7b: encoder
- 7c: arm
- 7d: force detector
- 7e: redundant joint
- 8: elongated part
- 9: flexible tube
- 10: channel
- 10A: channel distal part
- 10B: channel tube
- 10C: channel proximal part
- 11: distal opening
- 12: proximal opening
- 13: shape sensor for flexible tube
- 14: proximal end of insertion
- 15: drive part
- 16: connector
- 20: operation input device
- 21: master arm
- 22: input part
- 23a: master joint
- 23b: encoder
- 24: drive part
- 25: mode selector
- 26: switch
- 27: distance input mechanism
- 30: image processing device
- 35: display device
- 36: monitor
- 40: light source device
- 50: joint control device
- 51, 51A, 51B: controller
- 52: image data receptor
- 53: characteristic point setting part
- 54: distance measurement part
- 55: control target value setting part
- 56: distance comparator
- 57: determinator
- 58: orientation calculator
- 59: characteristic point extraction part
- 60: characteristic point position calculator
- 61: orientation-deviation calculator
- 62: distance-deviation calculator
- 63, 63A, 63B: correction amount calculator
- 64: operation command generator
- 65: drive signal generator
- 66: insertion-state detector
- 67: external force determinator
- 68: joint specifying part
- 70: storage
- 100: treatment instrument
- 101: insertion body
- 102: treatment instrument type information storage

## Claims

1. A medical system comprising:
a distal end having an end effector and a plurality of joints that changes an orientation and a position of the end effector;
a drive part configured to generate power for operating the joints; and
a controller configured to control the drive part,
wherein the controller includes:
a characteristic point setting part configured to extract a characteristic point of a target object and recognize the target object based on the characteristic point;
a distance measurement part configured to measure a distance between the end effector and the target object;
a correction amount calculator configured to calculate an amount of operation of the joints such that the end effector is directed to the target object and adjust the distance between the end effector and the target object to a predetermined distance; and
a drive signal generator configured to generate a drive signal for operating the drive part based on the amount of operation and output the drive signal to the drive part.

2. The medical system according to Claim 1, further comprising:
a force detector that is connected to the controller and detects a force which the joints receive from outside,
wherein the controller further includes an external force determinator configured to determine whether or not a force externally applied to the joints exceeds a predetermined value, and
the correction amount calculator generates a correction command for reducing the distance between the end effector and the target object to be shorter than the predetermined distance when it is determined by the external force determinator that the force exceeds the predetermined value.

3. The medical system according to Claim 1, further comprising:
a force detector that is connected to the controller and detects a force which the joints receive from outside,
wherein the distal end has three or more of the joints,
the controller further includes a joint specifying part that specifies a joint subjected to a force exceeding a predetermined value among the plurality of joints, and
the correction amount calculator generates a correction command for moving the joint specified by the joint specifying part such that the force applied to the joint specified by the joint specifying part is equal to or less than the predetermined value and the position and the orientation of the end effector are maintained.

4. The medical system according to Claim 1, wherein
the end effector has an imager that images the target object,
the characteristic point setting part recognizes the target object by using an image imaged by the imager, and
the correction amount calculator calculates an amount of operation of the drive part so that the target object is located at an image center of the imager.

5. The medical system according to Claim 4, wherein
the imager is capable of imaging a stereo image of the target object, and
the distance measurement part calculates a distance between the target object and the imager using the stereo image.

6. The medical system according to Claim 1, further comprising:
an operation part configured to give an operation command to the controller,
wherein the operation part includes:
a master arm including an input part corresponding to the end effector and a plurality of master joints corresponding to the joints and having a shape conforming to the distal end; and
a master drive part that is connected to the controller and controls an operation of the master joint,
the controller further includes an operation command generator configured to detect an amount of operation of the master joint and generate an operation command including an amount of operation of a corresponding joint, and
the drive signal generator outputs a drive signal to the master drive part so that the joint and the master arm maintain a similarity relationship.

7. The medical system according to Claim 1, wherein
the distal end has a channel through which a medical instrument can be inserted.

8. The medical system according to Claim 7, further comprising:
an insertion state determination mechanism provided in the channel so as to determine whether or not a treatment tool that can be passed through the channel is inserted through the channel;
an insertion state detector configure to obtain a second predetermined distance preset corresponding to a type of the treatment tool, based on a determination state by the insertion state determination mechanism; and
a control target value setting part configured to set a control target value of a distance between the end effector and the target object to the second predetermined distance,
wherein the correction amount calculator calculates the amount of operation of the joints so that the distance between the end effector and the target object becomes the second predetermined distance.

9. A method of operating a medical system including a distal end having an end effector disposed thereon, the method comprising:
a characteristic point recognition step of extracting a characteristic point of a target object and recognizing the target object based on the characteristic point;
a distance measuring step of measuring a distance between the end effector and the target object; and
a correction amount calculation step of calculating an amount of operation of joints so that the end effector is directed to the target object and the distance between the end effector and the target object becomes a predetermined distance.
